(19) Europäisches Patentamt
European Patent Office
Office européen des brevets

(11) **EP 4 610 248 A1**

(12) **EUROPEAN PATENT APPLICATION**

(43) Date of publication:
**03.09.2025 Bulletin 2025/36**

(21) Application number: **24161009.6**

(22) Date of filing: **01.03.2024**

(51) International Patent Classification (IPC):
***C07C 53/126*** (2006.01)    ***C07C 51/09*** (2006.01)
***B01J 29/00*** (2006.01)

(52) Cooperative Patent Classification (CPC):
(C-Sets available)
**C07C 51/09; B01J 23/44; B01J 29/40;
B01J 29/7007; B01J 35/19; C07C 51/36;
C07C 53/126;** B01J 35/00; B01J 35/615;
B01J 35/617                                    (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicants:
• **Cargill, Incorporated
Wayzata, MN 55391 (US)**
• **Universiteit Utrecht Holding B.V.
3584 CS Utrecht (NL)**

(72) Inventors:
• **BOS, Jelle Wouter
3584 CS Utrecht (NL)**
• **VOGT, Eelco Titus Carel
3584 CS Utrecht (NL)**
• **WIEDEMANN, Sophie Claude Catherine
Wayzata, 55391 (US)**
• **MOONEN, Raoul Charles Johan
Wayzata, 55391 (US)**
• **WECKHUYSEN, Bert Marc
3584 CS Utrecht (NL)**

(74) Representative: **V.O.
P.O. Box 87930
2508 DH Den Haag (NL)**

(54) **PRODUCTION OF A BRANCHED SATURATED FATTY ACID**

(57)    The invention is in the field of fatty acid production. Provided is a process for the production of a branched saturated fatty acid, comprising the steps of: a) providing a reactant mixture comprising one or more branched unsaturated fatty acids and one or more fatty acid lactones, b) contacting the reactant mixture with hydrogen in the presence of a hydrogenation catalyst and an acid catalyst; wherein the acid catalyst comprises zeotype material having: - an external surface area of 100 $m^2/g$ or more, measured using Arphysisorption, and - acid sites of intermediate strength and acid sites of low strength determined using temperature-programmed desorption of $NH_3$ at temperatures of 250-400 °C and 100-250 °C, respectively, wherein the ratio (acid sites of intermediate strength) : (acid sites of low strength) is 0.9 or more.

EP 4 610 248 A1

(52) Cooperative Patent Classification (CPC): (Cont.)

C-Sets
**C07C 51/09, C07C 53/126;**
**C07C 51/36, C07C 53/126**

**Description**

BACKGROUND OF THE INVENTION

The invention is in the field of fatty acid production. In particular, the invention is directed to a process for the production of a branched fatty acid.

Fatty acids are used for a wide range of applications. For instance, fatty acids are used in the food industry, as an ingredient in cosmetics and personal care products, and as lubricants. Industrially, fatty acids can be produced by hydrolysis of triglycerides found in vegetable or animal fats. The chain length and degree of saturation of the resulting fatty acids is strongly dependent on the feedstock.

Branched-chain fatty acids are a special class of fatty acids, in which one or more alkyl groups, typically methyl groups, are attached to the main aliphatic chain of the fatty acid. Most naturally occurring branched-chain fatty acids are saturated, although unsaturated branched may also occur in nature. Compared to their unbranched counterparts, branched-chain fatty acids typically have different physical properties which may be desirable, such a lower melting point. Saturated branched-chain fatty acids are typically more stable than their unsaturated counterparts, and are therefore particularly useful for a wide range of applications. For example, saturated branched-chain fatty acids (such as commercial grade "isostearic acid") are frequently used in demanding applications such as personal care products, lubricants and hydraulic fluids, because of its favorable properties, such as thermal and oxidative stability, long shelf life, low-temperature liquidity, and biodegradability.

Only a small fraction of naturally occurring fatty acids are branched-chain fatty acids. Therefore, more substantive amounts of branched-chain fatty acids are often produced from unbranched fatty acids.

For instance, isomerization and hydrogenation of unsaturated unbranched fatty acids may be performed in order to produce saturated branched fatty acids. In this process, isomerization of the unsaturated unbranched fatty acid yields a branched unsaturated fatty acid, which is subsequently hydrogenated into a branched saturated fatty acid. For instance, in order to produce isostearic acid, oleic acid can be isomerized into iso-oleic acid, followed by hydrogenation of iso-oleic acid to form isostearic acid.

During the isomerization reaction, which is typically performed at temperatures of 200-300 or even 250-300 °C in the presence of a solid isomerization (e.g. clay) catalyst, substantial amounts of unwanted by-products may be formed. Between the isomerization step and the hydrogenation step, byproducts are typically removed from the reaction mixture in order to improve purity of the final product. Removal of unwanted byproducts is typically performed using distillation, which effectively separates the unsaturated branched fatty acids from unwanted byproducts having a different boiling point, such as fatty acid oligomers.

However, other by-products are more difficult to remove using distillation. Fatty lactones, which may be formed by intramolecular esterification of the carboxylic acid group of an unsaturated fatty acid (*e.g.* γ-stearolactone is typically formed as byproduct during isomerization of oleic acid) are odorous and oxidatively labile by-products whose boiling points are too close to the boiling point of the corresponding branched fatty acids to be effectively removed using distillation. Therefore, removal of lactone impurities from the branched saturated fatty acid product is costly and energy consuming.

US3036099 describes hydrogenation of unsaturated fatty acids with lactone impurities, which is performed in the presence of an active clay. However, this process has some disadvantages. For instance, high reaction temperatures and/or long reaction times are needed in order to remove lactones to a sufficient extent. In addition to high costs and high energy consumption, these high temperatures and/or long reaction times can lead to a number of other problems, such as a further increase in formation of by-products, formation of colored compounds, and deactivation of the active clay and/or the hydrogenation catalyst.

Another downside related to the use of acidic clay is that it is typically very abrasive, and large amounts of clay are needed. Especially in combination with the high reaction temperature and long reaction times, this puts considerable strain on the equipment used for the reaction. Reaction vessels or conduits through which the reaction mixtures are transported need to be replaced regularly due to abrasive wear.

Another downside of active clays is that they are natural products of which the properties strongly depend on the location where they are mined and can only be modified to a small extent.

An object of the present invention is to provide a process for the production of a branched fatty acid in which one or more of the above-mentioned downsides are addressed.

Specifically, an object of the invention is produce branched fatty acids in a time- and/or energy-efficient manner.

Another object of the invention is to produce branched fatty acids while limiting the formation of by-products.

Another object of the invention is to provide a predictable process for producing branched fatty acids.

Another object of the invention is to provide a process for producing branched fatty acids that is less demanding on the equipment.

BRIEF SUMMARY OF THE INVENTION

The inventors have found that at least one of the downsides of the processes for the production of branched fatty acid known in the art can be solved by performing hydrogenation of the unsaturated branched fatty acid with lactone impurities in the presence of a zeotype material. It was found that in this way, saturated branched fatty acid can be produced with low amounts of lactone impurities, without the need for extensive purification after production. Surprisingly, it was found that zeotype materials with specific properties in terms of acidity and porosity are well-suited for removing lactone impurities during the production of saturated branched fatty acids. Specifically, zeotype materials which have a relatively high density of acidic sites of intermediate strength compared to the density of weak acid sites, and/or with a high external surface area, were found to be well-suited for producing branched saturated fatty acids from unsaturated unbranched fatty acids.

Zeotype materials with an external surface area of 100 m$^2$/g or more and a ratio (acid sites of intermediate strength) : (acid sites of low strength) of 0.9 or more were found to perform particularly well. Relative to known processes and the catalyst materials used therein, the use of such zeotype materials can result in improvements, such as reduced tendency to form lactone by-products, improved activity at the same or lower reaction temperatures, faster lactone conversion, lower amounts of catalyst needed, and/or less abrasion of equipment.

Without wishing to be bound by theory, the inventors believe that reduction of lactone in the final product is achieved, at least partly, because zeotype catalyst with the above-mentioned properties effectively catalyze the ring opening of the type of lactones that may be formed during isomerization of fatty acids, particularly with chain lengths that are abundant in nature (e.g., C12 or C22 as found in vegetable oils).

BRIEF DESCRIPTION OF THE DRAWINGS

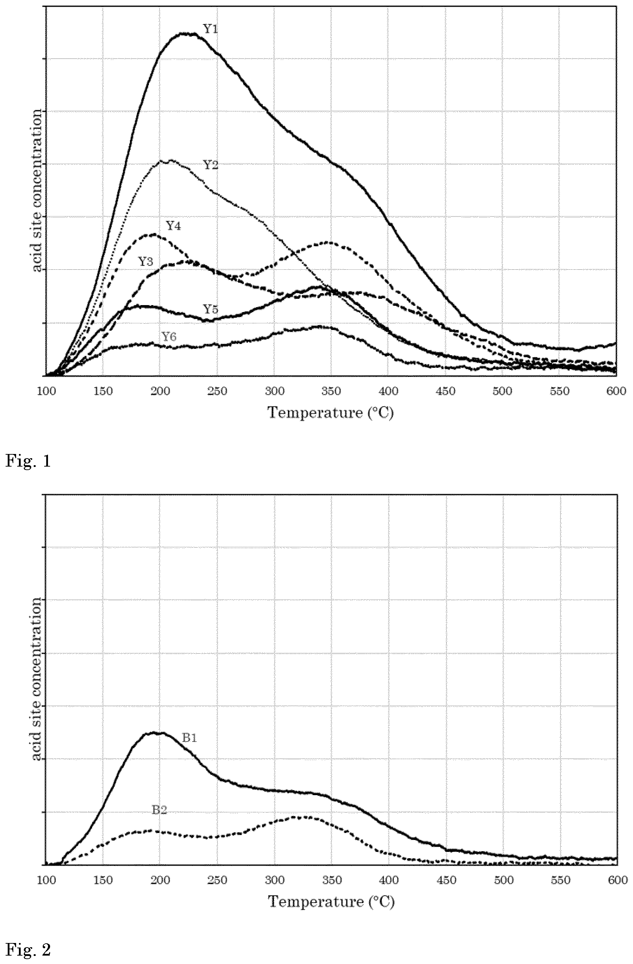

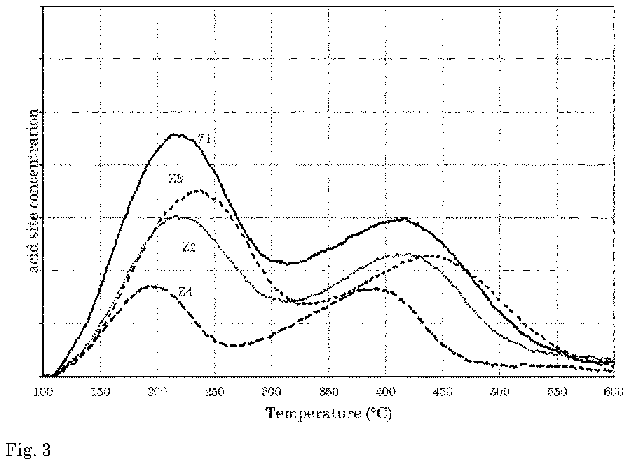

In figures 1-3, desorption curves for temperature-programmed desorption of NH$_3$ are shown for various zeotype samples.

DETAILED DESCRIPTION OF THE INVENTION

According to the invention there is provided a process for the production of a branched fatty acid.

As reactant, a mixture comprising one or more branched unsaturated fatty acids and one or more fatty acid lactones is provided. This mixture may be obtained by isomerization of one or more unbranched unsaturated fatty acids, which is typically used as a first step for converting unbranched unsaturated fatty acid into saturated branched fatty acids.

The process comprises contacting the mixture comprising one or more branched unsaturated fatty acids and one or more fatty acid lactones with hydrogen in the presence of a hydrogenation catalyst and an acid catalyst, thereby producing a saturated branched fatty acid.

In principle, the process of the invention can be performed using a reactant mixture comprising any amount of lactone impurities. The amount of lactones present in the reactant mixture may be dependent on the process that was used to obtain the reactant mixture, but will typically be 1.0% by weight or more relative to the entire reactant mixture, for instance up to 10 wt.%. In case the reactant mixture comprising one or more branched unsaturated fatty acids and one or more fatty acid lactones is obtained by isomerization of one or more unbranched unsaturated fatty acids, the amount of lactones depends on factors such as the type of catalyst used in the isomerization process, and whether the isomerization process is directed to polymeric or monomeric acids.

The reactant mixture typically comprises 50% or more by weight of the one more branched unsaturated fatty acids relative to the entire reactant mixture, depending on the on the process that was used to obtain the reactant mixture. Preferably, the mixture comprises 55 wt.% or more branched unsaturated fatty acids, for instance 65-99 wt.%, or 70-95 wt.%.

Fatty acid lactones, as used herein, refer to lactones that may be formed by intramolecular esterification of branched or unbranched fatty acids, typically of unsaturated fatty acids. Typically, lactones formed in this way are δ- or γ-lactones. Fatty acid lactones may have a hydrocarbon tail attached to the lactone ring, which tail can be branched or unbranched, and which tail can be saturated or unsaturated. The total number of carbon atoms in fatty acid lactones is typically the same as the number of carbon atoms in corresponding fatty acids (*e.g.*, γ-stearolactone can be formed from corresponding fatty acid oleic acid, both of which have 18 carbon atoms).

Unsaturated fatty acids, as used herein, refer to fatty acids having one or more carbon-carbon double bond in the carbon chain. Each unsaturated carbon-carbon bonds may individually be *cis*-unsaturated or trans-unsaturated.

Branched fatty acids, as used herein, refer to fatty acids having one or more alkyl groups attached to the main carbon chain.

The inventors believe that contacting fatty acid lactones with the acid catalyst results in ring-opening of the lactones, thereby producing unsaturated fatty acids. The unsaturated fatty acids (which may be branched or unbranched, depending on whether the lactone was branched or unbranched) produced by ring-opening of the lactones are subsequently hydrogenated, thereby further shifting the equilibrium towards lactone ring-opening.

The acid catalyst preferably comprises a zeotype material. Zeotype materials are crystalline materials with a well-defined micropore structure. The main classes of zeotype materials are silicoaluminate zeotype materials (*i.e.*, zeolites) and silico-aluminophosphate (SAPO) zeotype materials. An overview of different zeotype frameworks and their pore structures can be found on the website of the International Zeolite Association: www.iza-structure.org.

Preferably, the zeotype material has a high external surface area. As used herein, external surface area of the zeotype material refers to the total accessible surface area of the material, with the exception of the micropore surface area. External surface areas of a material can be determined using methods known in the art, such as low-temperature physisorption of a small probe molecule, such as $N_2$ or Ar. Based on such physisorption data, the total surface area can be determined, *e.g.* according to the Brunauer-Emmett-Teller (BET) theory, and micropore surface area can be determined using the t-plot method. Preferably, the external surface area of the zeotype material is determined according to ISO 9277.

In case of zeotype materials, catalytic activity and selectivity are often believed to be strongly related to catalytically active sites present inside the zeotype's well-defined micropore structure. However, surprisingly, in the process according to the present invention, the micropore surface area does not determine the suitability of a zeotype material as acid catalyst.

Preferably, the zeotype material has an external surface area $S_{ext}$ of 100 $m^2$/g or higher, more preferably 110 $m^2$/g or higher, or 120 $m^2$/g or higher, or even 125 $m^2$/g or higher, such as 130 $m^2$/g or higher or 135 $m^2$/g or higher. The external surface area may even be as high as 800 $m^2$/g but will typically be lower than that, for instance in the range of 100-500 $m^2$/g, or 120-400 $m^2$/g.

Additionally or alternatively, the zeotype material preferably has a specific ratio between the density of acid sites of different strengths. The acidity of zeotype materials may conveniently be determined using chemisorption techniques, such as temperature-programmed desorption of an alkaline probe molecule, for instance $NH_3$. Based on the amount of probe molecule that desorbs from the material's surface at a certain temperature, the acid site density and strength of acid sites of a zeotype material can be determined. As used herein, the term acid sites of low strength (or weak acid sites) refer to acid sites from which $NH_3$ desorbs at temperatures in the range of 100-250 °C. Acid sites of intermediate strength refer to acid sites from which $NH_3$ desorbs at temperatures in the range of 250-400 °C. Acid sites of high strength refer to acid sites from which $NH_3$ desorbs at temperatures in a range of 400-600 °C. The ratio between acid sites of different strength, for instance the ratio (acid sites of intermediate strength) : (acid sites of low strength) is calculated by dividing the density of acid sites of intermediate strength by the density of acid sites of low strength according to the classification above, and as measured by $NH_3$-TPD.

In case of zeotype materials, catalytic activity is often believed to be related to the presence of strongly acidic sites. However, it was found that for the process according to the invention, activity in lactone conversion is related to the ratio between acid sites of intermediate strength and acid sites of low strength. The zeotype material preferably has acid sites of intermediate strength and acid sites of low strength, wherein the ratio (acid sites of intermediate strength) : (acid sites of low strength) is 0.9 or more. The ratio (acid sites of intermediate strength) : (acid sites of low strength) may be for instance even be as high as 3.0, but will typically be lower than that, for instance in the range of 0.9-2.0, or 0.9-1.5.

Preferably, the zeotype material has both the external surface area and the ratio between acid sites of intermediate strength and acid sites of low strength as described above. Without wishing to be bound by theory, the inventors believe that zeotype materials with these properties provide a sufficient number of acid sites having sufficient strength, specifically on surfaces of the zeotype material that are accessible for fatty acid lactones. Too many acid sites of low strength relative to the amount of acid sites of intermediate strength may be indicative for blocking of meso- and macropores by extra-framework species, thereby limiting access of the lactones to the active sites, whereas acid sites of high strength may be predominantly located inside micropores, which are also difficult to access for larger molecules.

Zeolites with the properties described above in terms of external surface area and acidity can be obtained commercially. Additionally or alternatively, the preferred properties can be achieved by treating commercially obtained or as-synthesized zeotype materials. For instance, treating a zeotype material using steam at elevated temperatures (*e.g.* in a range of 400-700 °C) and/or desilication of zeotype material using an alkaline solution may be performed in order to create meso- or macropores, thereby increasing the external surface area of a zeotype material. Alternatively or additionally, washing a zeotype material using an acidic solution can be performed in order to remove extra-framework species that may be present in the pores. Substantial amounts of extra-framework species may for instance be present as a result of the above-described steam treatment or desilication as described above. Since extra-framework species are often weakly acidic, removing these species by acid-washing may influence the external surface area and/or the ratio of acid sites of different strengths of a zeotype material.

As described above, suitability of the zeotype material for the process of the invention is related to external surface area and acidic properties of the material. In addition, the zeotype's micropore structure may also contribute to suitability of the material for the process of the invention. Zeotype frameworks with large pores, *i.e.* micropores of which the size is determined by 12-membered or larger rings may be preferred, because of their accessibility to fatty acids with a long carbon chain. Examples of large-pore zeotype frameworks include FAU, MOR, and BEA. However, medium- and small-pore zeotype frameworks with micropores of which the size is determined by 10- or 8-membered rings, such as MFI, FER, and CHA may also be suitable.

Step b) of contacting the reactant mixture with hydrogen in the presence of a hydrogenation catalyst and an acid catalyst may be performed at temperatures in a range of 120-350 °C. In processes known in the art, temperatures of 230 °C or higher are needed in order to reach sufficient lactone removal. Using the zeotype material as described above, lactone can already be removed efficiently at lower temperatures, for instance in the range of 120-250 °C, or 150-230 °C. Preferably, step b) is performed at a temperatures lower than 230 °C, more preferably 225 °C or lower 220 °C or lower, or even 210 ° or lower, for instance 180-230 °C, or 190-220 °C.

Step b) of contacting the reactant mixture with hydrogen in the presence of a hydrogenation catalyst and an acid catalyst may be performed at a gauge pressure in a range of 5-100 bar, preferably 10-50 bar.

The zeotype material may be a silicoaluminate zeolite material. Si/Al ratios that are suitable for the process of the invention are 2-300, preferably 5-100. The zeolite Si/Al ratio is loosely related to the total acid site density and hydrophobicity of silicoaluminate zeolite materials. A moderate degree of hydrophobicity and hydrophilicity is preferred for the ring opening of lactones with a long carbon tail. If the Si/Al ratio is too high, the total acid site density may become too low and/or the zeolite may become too hydrophobic, whereas a too low Si/Al ratio may result in decreased physical strength and/or a too high hydrophilicity of the zeolite.

Alternatively, the zeotype material may be a silico-aluminophosphate (SAPO) material. In case of a SAPO material, the (Al+P)/Si ratio is related to acid site density and hydrophobicity in a similar manner as the Si/Al ratio in case of silicoaluminate materials. The zeotype material may be a silicoaluminate zeolite material. (Al+P)/Si ratios that are suitable for the process of the invention are 2-300, preferably 5-100.

The acid site density of the zeotype material may suitably be in the range of 0.1-1.5 mmol/g. Preferably, the acid site density is in the range of 0.2-1.0 mmol/g.

Step b) of contacting the reactant mixture with hydrogen in the presence of a hydrogenation catalyst and an acid catalyst is preferably performed for a short time, thereby preventing excessive energy consumption and reducing the unwanted formation of by-products. Using the process as described herein, shorter reaction times are needed compared to prior art processes to achieve the same or a higher degree of lactone removal, using the same amount of acid catalyst. Preferably, step b) is performed for a duration of 7 hours or less, more preferably 6 hours or less, such as 4 hours or less, or 0.5-2.5 hours. The reaction time needed to achieve enough lactone removal may depend on the type of reactor used. In embodiments, step b) is performed in a continuous reactor, preferably for a duration of 7 hours or less, more preferably 6 hours or less, such as 1-5 hours, or 0.5-4 hours. Alternatively, step b) may be performed batchwise, preferably for a duration of 4 hours or less, more preferably 3 hours or less, such as 0.5-2.5 hours.

Additionally or alternatively, using the process as described herein, the same or a higher degree of lactone removal can be achieved while using lower amount of acid catalyst compared to prior art processes. Preferably, step b) is performed with 1.0 % or less by weight of acid catalyst relative to the total weight of the reactant mixture, more preferably 0.80 wt.% or less, or 0.65 wt.% or less.

Branched saturated fatty acids of different chain lengths can be produced, and their counterpart lactones can be removed using the process as described herein. Preferably, the one or more branched unsaturated fatty acids and one or more fatty acid lactones present in the reactant mixture have a carbon chain with a length in the range of 12-22 carbon atoms, preferably 16-20 carbon atoms. Fatty acids having a C18 carbon chain, e.g. iso-oleic acid and its counterpart $\gamma$-stearolactone, are particularly preferred.

The hydrogenation catalyst may comprise one or more metals selected from the group consisting of nickel, palladium, platinum, iridium, rhodium, and ruthenium. For instance the hydrogenation catalyst may be in the form of metal particles on a support.

Preferably, the product mixture obtained in step comprises 0.5 % by weight or less of fatty acid lactones, relative to the total weight of the product mixture. More preferably, the product mixture comprises 0.3 wt.% or less, or even 0.2 wt.% or less of fatty acid lactones.

The process as described herein may be preceded by contacting one or more unbranched unsaturated fatty acids with an isomerization catalyst. Isomerization is typically performed at elevated temperatures of *e.g.* in a range of 200-350 °C in the presence of a clay (*e.g.* bentonite clay) catalyst.

As described above, this will typically result in a mixture comprising one or more branched unsaturated fatty acids and one or more fatty acid lactones. Optionally, the mixture obtained by isomerization of unbranched unsaturated fatty acids may be purified in order to remove impurities from the mixture prior to subjecting the mixture to step b). Impurities that can be removed include for instance fatty acid oligomers, which can for instance be removed from the mixture using distillation.

EXAMPLES

**Example 1**

Different zeolite samples were prepared for testing in a model reaction. Zeolite samples were commercially obtained. Zeolite Y and ZSM-5 samples were obtained from Zeolyst (The Netherlands), and zeolite beta samples were obtained from Alfa Aesar (United States).

Some samples were used without further modification. Other samples, which were provided by the supplier in ammonium form, were calcined in air for at least 5 h at temperatures of 500-550 °C to obtain the samples in proton form.

In addition, some samples underwent subsequent treatments in order to modify their properties. Sample B2 was obtained by treatment of B1 as follows: after calcination as described above, the sample was steamed for 24 h at 500 °C under a 80% humidity atmosphere and 45 mL/min nitrogen flow. Lastly, the sample was acid washed for 2 h at 60 °C in 2M HNOs and washed with demineralized water.

Sample Z2 was obtained by treatment of Z1 as follows: after calcination as described above, the sample was desilicated for 30 min at 65 °C using 0.8 M NaOH, washed until neutral and dried. Subsequently, the sample was washed with 0.05 M $H_2SO_4$ for 5 h at 65 °C, then washed until neutral and dried.

In Table 1, an overview of the zeolite samples is provided.

**Table 1**

| Sample no. and name | framework type | description of treatment |
|---|---|---|
| Y1: CBV300 | Zeolite Y (FAU) | commercial, calcined to obtain proton form |
| Y2: CBV400 | Zeolite Y (FAU) | commercial |
| Y3: CBV600 | Zeolite Y (FAU) | commercial |
| Y4: CBV712 | Zeolite Y (FAU) | commercial, calcined to obtain proton form |
| Y5: CBV720 | Zeolite Y (FAU) | commercial |
| Y6: CBV760 | Zeolite Y (FAU) | commercial |
| B1: BH | Zeolite beta (BEA) | commercial, calcined to obtain proton form |
| B2: BH-HT24-Al2 | Zeolite beta (BEA) | based on sample B1 (calcined), subsequently steamed and acid washed |
| Z1: CBV2314 | ZSM-5 (MFI) | commercial, calcined to obtain proton form |
| Z2: CBV2314-NaOH-H2SO4 | ZSM-5 (MFI) | based on sample Z1 (calcined), subsequently desilicated and acid washed |
| Z3: CBV3024E | ZSM-5 (MFI) | commercial, calcined to obtain proton form |
| Z4: CBV8014 | ZSM-5 (MFI) | commercial, calcined to obtain proton form |

**Example 2**

Specific surface areas of the zeolite samples were analysed using physisorption of argon at 87K using a 3P Sync400 physisorption machine. Prior to the measurements, samples were degassed under vacuum at 400 °C for 16h. Specific surface areas were determined according to ISO 9277.

The total surface area $S_{tot}$ was determined according to the Brunauer-Emmett-Teller (BET) theory. The micropore surface area $S_{micro}$ was determined via the empirical t-plot method in the range of 0.43-0.65 nm using the Harkins Jura method. The external surface area $S_{ext}$ was defined as $S_{meso} + S_{macro}$, where $S_{meso} + S_{macro} = S_{tot} - S_{micro}$.

In addition, the zeolite samples were characterized using temperature-programmed desorption of ammonia ($NH_3$-TPD). $NH_3$-TPD measurements were performed on a Micromeritics Autochem II Chemisorption Analyzer equipped with a TCD detector. Prior to adsorption of ammonia, zeolites samples of 80-120 mg were heated to 600 °C with a rate of 5 °C/min and dried for 60 min at 600 °C to remove any adsorbed species from the zeolite. Then an excess of ammonia was pulsed, to fully saturate the sample. Lastly the zeolite was heated to 600 °C at a heating rate of 5 °C/min and ammonia was left to desorb at 600 °C for a further 30 minutes.

NH3-TPD profiles for the zeolite Y, zeolite beta, and ZSM-5 samples are shown in figure 1, 2 and 3, respectively. The acid site densities were categorized by acid strength, based on the temperature regions 100-250 °C, 250-400 °C and 400-600 °C.

The ratio (acid sites of intermediate strength) : (acid sites of low strength) was determined by dividing the intermediate strength acid site density by the low strength acid site density.

For sample B2, Si/Al ratio was determined using ICP-OES analysis performed by Mikrolab Kolbe (Germany). For the commercial samples, Si/Al ratios provided by the by the commercial supplier are listed.

Characteristics of the zeolite samples are summarized in Table 2.

**Table 2**

| Sample no. | Si/Al ratio | acid site density (mmol/g) | | | | ratio IT/LT | surface area (m²/g) | | |
|---|---|---|---|---|---|---|---|---|---|
| | | total | LT(100-250 °C) | IT(250-400 °C) | HT(400-600 °C) | | $S_{tot}$ | $S_{micro}$ | $S_{ext}$ |
| Y1 | 2.55 | 1.56 | 0.67 | 0.68 | 0.22 | 1.01 | 771 | 710 | 61 |
| Y2 | 2.55 | 0.77 | 0.41 | 0.30 | 0.06 | 0.72 | 726 | 636 | 90 |
| Y3 | 2.6 | 0.54 | 0.20 | 0.23 | 0.12 | 1.13 | | | n.d.* |
| Y4 | 6 | 0.68 | 0.26 | 0.31 | 0.10 | 1.17 | 748 | 613 | 136 |
| Y5 | 15 | 0.45 | 0.16 | 0.22 | 0.07 | 1.35 | 807 | 611 | 196 |
| Y6 | 30 | 0.42 | 0.15 | 0.21 | 0.06 | 1.38 | 788 | 600 | 188 |
| B1 | 12.5 | 0.60 | 0.30 | 0.24 | 0.06 | 0.80 | 510 | 320 | 190 |
| B2 | 55 | 0.25 | 0.10 | 0.14 | 0.01 | 1.45 | 521 | 311 | 210 |
| Z1 | 23 | 1.16 | 0.47 | 0.40 | 0.29 | 0.85 | 351 | 314 | 37 |
| Z2 | n.d.** | 0.81 | 0.30 | 0.28 | 0.23 | 0.94 | 508 | 214 | 294 |
| Z3 | 30 | 0.94 | 0.34 | 0.31 | 0.29 | 0.89 | 528 | 460 | 68 |
| Z4 | 80 | 0.46 | 0.17 | 0.19 | 0.10 | 1.11 | 466 | 383 | 73 |

*not measured, predicted value based on literature reference: ca. 83 m²/g
**not measured, predicted value based on the measured acid site density relative to Z1: > 23

**Example 3**

Suitability of the zeolite samples for the process according to the invention was assessed using a model reaction in which a mixture of iso-oleic acid and iso-stearic acid is contacted with the zeolite sample, as well as hydrogen gas in the presence of a hydrogenation catalyst.

Prior to catalytic testing, the zeolite powders were pressed into pellets, crushed, and lastly sieved in a size fraction of 212 - 425 μm. 200 g of a fatty acid mixture of iso-oleic acid and isostearic acid in a weight ratio of 40 : 60 was prepared and added to a stirred autoclave reactor. Due to impurities in the iso-oleic acid, the γ-stearolactone concentration in the mixtures ranged between 2.29 and 2.42 wt.%, depending on the iso-oleic acid feedstock used. Afterwards, 0.5 g of the sieved zeolite and 0.25 g of Pd/C were added to the reactor. The system was then closed and flushed three times with $N_2$ and $H_2$ to remove any oxygen molecules. The reactor was stirred at 600 rpm and a $H_2$-pressure of 15 bar was applied. The temperature was increased by 2 °C/min until a temperature of 190 °C was reached. Then, the ramp was increased to 4 °C/min and the pressure was increased to 20 bar. Once a temperature of 230 °C was reached, the pressure was finally set to 25 bar. Samples were taken at regular intervals to be able to follow the ring-opening and hydrogenation reactions over time.

Gamma lactone concentration was analyzed using FT-IR using a single bounce ATR accessory, temperature-controlled at 35.0 °C. The carbonyl vibration characteristic of the gamma lactone group at wavelength 1784 cm$^{-1}$ was used to quantify the concentration, based on external calibration.

The activity of the isomerization catalyst in γ-stearolactone conversion in wt.%lactone/(h×gcat) was determined using the following formula:

$$\text{Activity} = (w_{feed} - w_{end})/(t \times m),$$

wherein $w_{end}$ is the weight percentage lactone of the final sample, $w_{feed}$ is the weight percentage lactone in the beginning of the reaction (feedstock concentration), t is the reaction time in hours and m is the weight of catalyst added in grams.

The acidity-scaled activity was calculated by normalizing the activity with respect to the total acid site density of the samples, based on an acid site density of 1 mmol/gcat.

As a comparative example, the same experiment was also performed using 0.5 g of clay (Taiko Omega 1G) as acid catalyst instead of the zeolite samples.

Results of the catalytic tests are shown in Table 3.

**Table 3**

| Sample no. | lactone carbon chain length | activity wt.% lactone/(h×gcat) | normalized activity wt.% lactone/(h×mmol) |
|---|---|---|---|
| Y1 | C18 | 0.82 | 0.53 |
| Y2 | C18 | 0.93 | 1.21 |
| Y3 | C18 | 0.84 | 1.56 |
| Y4 | C18 | 1.89 | 2.78 |
| Y5 | C18 | 1.52 | 3.38 |
| Y6 | C18 | 4.55 | 10.83 |
| B1 | C18 | 0.95 | 1.58 |
| B2 | C18 | 2.32 | 9.25 |
| Z1 | C18 | 0.77 | 0.66 |
| Z2 | C18 | 4.64 | 5.73 |
| Z3 | C18 | 0.76 | 0.8 |
| Z4 | C18 | 0.68 | 1.48 |
| clay | C18 | 0.55 | |

## Example 4

In addition to the experiments of example 3 in which a mixture of iso-oleic acid and isostearic acid containing C18 lactone (*i.e.,* γ-stearolactone) was used, experiments were also performed on mixtures containing lactone with a C12 carbon chain and lactone with a C22 carbon chain, respectively.

The experimental conditions were the same as described above in example 3, except that a different reactant mixture was added to the stirred autoclave reactor instead of the mixture of iso-oleic acid and isostearic acid. Zeolite sample Y6 was used as acid catalyst.

For C12 lactone, 200 g of a mixture of dodecalactone and isostearic acid in a weight ratio of 10 : 90 was prepared and added to the stirred autoclave reactor.

For C22 lactone, 200 g of a fatty acid mixture of iso-erucic acid and isostearic acid in a weight ratio of 40 : 60 was prepared and added to the stirred autoclave reactor. Due to lactone impurities in the iso-erucic acid, the C22 lactone concentration in the mixture was ca. 1.75 wt.%.

As a comparative example, the experiment with C12 lactone was also performed using 0.5 g of clay (Taiko Omega 1G) as acid catalyst instead of zeolite sample Y6.

Results are shown in Table 4.

**Table 4**

| Sample no. | lactone carbon chain length | activity wt.%lactono/(h×gcat) |
|---|---|---|
| Y6 | C12 | 10.20 |
| clay | C12 | 3.95 |
| Y6 | C22 | 1.22 |

## Claims

1.  Process for the production of a branched saturated fatty acid, comprising the steps of:

    a) providing a reactant mixture comprising one or more branched unsaturated fatty acids and one or more fatty acid lactones,

b) contacting the reactant mixture with hydrogen in the presence of a hydrogenation catalyst and an acid catalyst;

wherein the acid catalyst comprises zeotype material having

- an external surface area of 100 m$^2$/g or more, measured using Arphysisorption, and
- acid sites of intermediate strength and acid sites of low strength determined using temperature-programmed desorption of NH$_3$ at temperatures of 250-400 °C and 100-250 °C, respectively, wherein the ratio (acid sites of intermediate strength) : (acid sites of low strength) is 0.9 or more.

2. Process according to claim 1, wherein step b) is performed at a reaction temperature in a range of 120-300 °C, preferably 150-230 °C.

3. Process according to claim 1 or 2, wherein step b) is performed at a gauge pressure in a range of 5-100 bar, preferably 10-50 bar.

4. Process according to any one of the preceding claims, wherein the zeotype material is zeolite material.

5. Process according to claim 4, wherein the zeolite material has a Si/Al ratio of 2-300, preferably 5-100.

6. Process according any one of claims 1-3, wherein the zeotype material is silico-aluminophosphate material.

7. Process according to claim 6, wherein the silico-aluminophosphate material has an (Al+P)/Si ratio of 2-300, preferably 5-100.

8. Process according to any one of the preceding claims, wherein the zeotype material has an acid site density of at least 0.1 mmol/g, preferably in a range of 0.2-1.0 mmol/g, measured using temperature-programmed desorption of NH$_3$.

9. Process according to any one of the preceding claims, wherein step b) is performed for a duration of 4 hours or less.

10. Process according to any one of the preceding claims, wherein the one or more branched unsaturated fatty acids and one or more fatty acid lactones present in the reactant mixture have a C12-C22 carbon chain.

11. Process according to any one of the preceding claims, wherein the hydrogenation catalyst is a supported metal catalyst.

12. Process according to any one of the preceding claims, wherein the hydrogenation catalyst comprises one or more metals selected from the group consisting of nickel, palladium, platinum, iridium, rhodium, and ruthenium.

13. Process according to any one of the preceding claims, wherein the product obtained in step b) is a product mixture comprising 0.5 % by weight or less of fatty acid lactones relative to the total weight of the product mixture.

14. Process according to any one of the preceding claims, preceded by:

i) contacting one or more unbranched unsaturated fatty acids with an isomerization catalyst,
ii) optionally, removing fatty acid oligomers from the mixture obtained in step i);

thereby forming the reactant mixture comprising one or more branched unsaturated fatty acids and one or more fatty acid lactones.

Fig. 1

Fig. 2

Fig. 3

Europäisches
Patentamt

European
Patent Office

Office européen
des brevets

# EUROPEAN SEARCH REPORT

**Application Number**

EP 24 16 1009

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | US 3 036 099 A (BARRETT FRED O ET AL) 22 May 1962 (1962-05-22) ----- | 1-14 | INV. C07C53/126 C07C51/09 B01J29/00 |
| | | | **TECHNICAL FIELDS SEARCHED (IPC)** |
| | | | C07C B01J |

The present search report has been drawn up for all claims

| Place of search | Date of completion of the search | Examiner |
|---|---|---|
| The Hague | 2 September 2024 | Menchaca, Roberto |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
    .......................................................................
& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 16 1009

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2024

| Patent document cited in search report | Publication date | Patent family member(s) | Publication date |
|---|---|---|---|
| US 3036099 A | 22-05-1962 | NONE | |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3036099 A